# EUROPEAN PATENT SPECIFICATION

(11) **EP 1 968 992 B1**
(45) Date of publication and mention of the grant of the patent: **09.09.2015**
(21) Application number: 07717253.4
(22) Date of filing: 05.01.2007
(51) Int. Cl.: C07H 1/00, C07H 13/06

(54) **PRODUCTION OF SUGAR ESTERS FROM VINYL FATTY ACID ESTERS**
HERSTELLUNG VON ZUCKERESTERN AUS VINYLFETTSÄUREESTERN
PRODUCTION D' ESTERS DE SUCRE A PARTIR D' ESTERS D' ACIDE GRAS VINYLIQUE

(30) Priority: 06.01.2006 US 757097 P
(43) Date of publication of application: 17.09.2008
(73) Proprietor: Comstock, Bob, Singapore 26695 (SG)
(72) Inventor: WANG, Mei, Yin, Singapore 530363 (SG)
(74) Representative: Fiener, Josef
(86) International application number: PCT/US2007/060140
(87) International publication number: WO 2007/082149

(56) References cited:
- GB-A- 890 083
- JP-A- 2006 265 544
- US-B1- 6 489 468

## Description

### Background of the Invention

### Area of the Art

This invention relates to a process for producing sugar esters with high efficiency and high purity.

### Description of the Related Art

Starting around the end of the Second World War "natural" surfactants such as soaps (alkali metal salts of fatty acids) were increasingly replaced with petroleum-derived "detergents." However, most of these materials were not suitable for use in food and drug products. In the search for surfactants suitable for food and drug use the sugar detergents or surfactants-namely fatty acid esters of sugars and sugar derivatives were discovered. Because of the relatively large number of natural sugars and the variety of possible fatty acids, a very large number of different sugar detergents are possible.

Sugar esters can be classified both by the type of sugar and also by the chain length of the fatty acid esterified to the sugar. Sucrose, glucose, maltose and other sugars have been reacted with a variety of fatty acids including stearic acid (18 carbons), oleic acid (18 carbons), palmitic acid (16 carbons), myristic acid (14 carbons) and lauric acid (12 carbons). Sucrose esters are surfactants or emulsifiers that have several desirable properties for use in the food industry. These include the ability to produce excellent emulsification of oils and water, anti-bacterial properties, freezing damage protection of dough and baked breads, complex formation with starch, and even preservation of fresh fruit. One of the advantages of sugar esters for these tasks is that both the hydrophilic portion of the molecule (the sugar) and the lipophilic portion of the molecule (the fatty acid) can be readily varied thus providing a wide variety of different compounds each having slightly different properties.

Methods for esterifying sugars with vinylesters are disclosed in US 890,089 and US 6,489,468.

Probably the most common sugar detergents are those based on sucrose (table sugar) which is actually a disaccharide consisting of one molecule of the six carbon aldo-sugar glucose linked to a molecule of the five carbon keto-sugar fructose. When sucrose and fatty acid are reacted to form an ester there are eight positions (four on each of the constituent sugars) on the sucrose molecule where the fatty acid can be esterified. Typically, monoesters, diesters and triesters are formed with only a small quantity of higher esters probably because of steric hindrance. When the reaction is with a single fatty acid, such as lauric acid, analysis shows that there are many different esters that form, typically 2-4 different monoesters and up to 6-8 different diesters. If fatty acid mixtures are used as starting materials, the products are even more complex. It will readily be appreciated that the chain length (as well as degree of saturation) of the fatty acid can have significant effects on the properties of the resulting sugar ester. Longer chain fatty acids produce esters that are solid or semi-solid at room temperature. Longer chain esters are also less readily soluble in water. The hydrophilic properties of the amphiphilic surfactant molecules are largely contributed by the sugar moiety while the hydrophobic properties are contributed by the fatty acid moiety with longer chain length acids being increasingly hydrophobic.

The United States Food and Drug Agency (FDA) has set standards for sugar detergents in foods, drugs and cosmetics. The food additive standards are found at 21 CFR 172.859, pages 95-96. The standards set requirements for the composition of the sucrose esters which must be 80% or more by weight of mono, di and tri esters produced from edible fatty acids (animal fats and vegetable oils) with less than 5% by weight free sucrose. In addition only "safe" organic solvents may be used in the synthesis of the esters and the residue levels of these solvents are controlled by the standards.

Perhaps as a result of these standards, there are only a few commercial sources of these materials that meet the Food and Drug Administration requirements. The sugar esters have been produced in Japan for over 50 years so that several Japanese companies, one being Ryoto Sugar Esters made by the Mitsubishi-Kasai Food Company, make products meeting the FDA criteria.

The classic method for the production of the sucrose esters is best illustrated by the production of sucrose stearate. In the early processes dimethyl formamide (DMF) was used as the solvent. The methyl ester of the fatty acid was made and this methyl ester was reacted with sucrose in the DMF in the presence of a catalyst such as potassium carbonate for 4-6 hours at 83-95.°C. Typical recipes (all are parts by weight) used 30 to 127 parts sucrose to 30 parts methyl stearate, 2 parts potassium carbonate and 300 parts solvent. These methods achieved high yields of 60-75% by weight. The basic problem has been in purifying the reaction mixture to yield a material that meets FDA standards especially since DMF is toxic. In addition, the reaction products are highly viscous containing partially decomposed sugars and solvents.

These problems led to a series of attempted improvements. Due to concerns about the use of DMF, Foster D. Snell developed a new method using dimethyl sulfoxide (DMSO) instead of DMF as disclosed in U.S. Pat. No. 3,480,616. Another example is found in U.S. Pat. No. 3,644,333 to Osipow et al which tried to eliminate the use of an organic solvent by using an emulsion. In this process, sucrose was mixed with methyl stearate and sodium stearate and a significant amount of previously prepared sucrose ester. Potassium carbonate was used as the catalyst, and water was used as the solvent. However, the yields are low, on the order of 30-35% by weight. Further, there are considerable waste and degraded products so that the desired product was much more difficult to remove from the reaction mixture.

Alternative methods have been described by Feuge. For example, in U.S. Pat. No. 3,714,144, he uses the sodium, potassium or lithium soap of the fatty acid in a molten sugar solution. The reaction proceeds for 2-20 minutes under vacuum at 170-190°C. Again, the product is made in very low yield and the destroyed sugar and alkali metals are very difficult to remove. To help with the production of the esters, several schemes have been devised to purify the reaction mixtures. For example, Kea et al in U.S. Pat. No. 4,710,567 teach the use of the addition of an aqueous salt solution to help purify the ester followed by a three phase separation.

A number of patents assigned to Dai-Ichi Kogyo Seiyaku Co. Ltd. (Kyoto, Japan) covering sucrose ester synthesis were granted between 1990 and 1991. In particular, U.S. Pat. Nos. 4,898,935, 4,996,309, 4,995,911, 5,011,922, and 5,017,697 all disclose methods for reacting, extracting and purifying sucrose fatty acid esters as produced using chemical catalysis.

### Summary of the Invention

The present invention is a process for synthesizing sugar esters of fatty acids using vinyl esters of fatty acids. By sugar we mean any of the naturally occurring sugars found primarily in plant sources. These include, for example, sucrose, ribose, mannose, glucose, fructose, xylose, arabinose, lactose, melibiose, galactose, mannose, raffinose and cellobiose. Appropriate fatty acids are those found in edible fats, waxes and oils-again primarily of vegetable origin. The useful fatty acids include, for example, hexanoic, octanoic, decanoic, lauric, myristic, myristoletic, palmitic, stearic, oleic, ricinoleic, linoleic, linolenic, arachidic, eicosenoic, behenic, and erucic acids. The inventive process combines a solution of an appropriate sugar in an appropriate organic solvent with a metal carbonate catalyst and a vinyl ester of a fatty acid as an acyl source for esterification. Suitable organic solvents are those solvents that show at least slight solubility of the sugar in question. In addition, because these sugar esters may be destined for food use, only FDA approved solvents can be used. Such solvents include dimethyl sulfoxide, methyl ethyl ketone, ethyl acetate, and isobutanol (2-methyl-1-propanol). The vinyl ester sugar mixture is reacted at a temperature between about 30°C and about 90°C for a time between about five minutes and twenty four hours. During the reaction the fatty acid groups esterify the sugar and acetaldehyde is released as a byproduct. Because acetaldehyde is reasonably volatile, the reaction can advantageously be conducted under reduced atmospheric pressure so that the acetaldehyde is removed from the reaction mixture, thereby "pulling" the reaction forward. At the end of the reaction time the product is primarily sugar monoester with a relatively low level of unconsumed vinyl ester if the starting ratio of sugar to vinyl ester is between about 2:1 and about 8:1.

### Detailed Description of the Invention

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide a method of producing sucrose esters wherein the fatty acids are provided in the form of vinyl esters.

Production of sucrose monostearate using vinyl esters of fatty acids

Because sucrose monoesters are considered to be more valuable than the other esters, the inventive reaction shown here has been optimized to produce the highest monoester content instead of the highest reaction yield.

Sucrose is first dissolved in DMSO at 60°C to a concentration of about 400 mM. To limit coloration of the reaction mixture, potassium carbonate is added to this sucrose solution at an amount of 1.5g/L and stirred for about 30 min to activate the sucrose molecules. After about 30 min, the undissolved potassium carbonate is removed by either decanting or filtration and vinyl stearate is added such that its final concentration is about 100 mM. With the addition of the vinyl fatty acid ester the reaction begins and is allowed to proceed for about 15 min. The reaction can be monitored by measuring the amount of remaining vinyl fatty acid ester. When this level falls to no more than about 10% by weight of the starting amount, the reaction can be considered to be complete. Using the above reaction conditions the monoester content, measured as a percentage of total ester content, post reaction is about 90%, and the calculated reaction yield based on vinyl stearate is about 90%. The acid value post reaction is less than 1.

### Purification of sucrose monostearate

The DMSO can be recovered from the reaction mixture by vacuum distillation. Brine (sodium chloride) is added to the post distillation mixture to effect emulsification of the sucrose stearate and vinyl stearate. The unreacted sucrose and potassium carbonate dissolve in the brine solution. To recover the sucrose stearate, especially sucrose monostearate, the emulsion is then broken by adding an organic solvent, for example, isobutanol. The organic solvent separates from the aqueous brine to form two separate liquid phases. One phase comprises organic solvent (isobutanol), sucrose ester (stearate) and unreacted vinyl ester. The other phase comprises of water, unreacted sucrose and potassium carbonate. After separation is complete, the isobutanol phase is decanted from the aqueous layer and subjected to vacuum distillation to recover the isobutanol. After the isobutanol is removed, the crude product comprises of sucrose stearate, vinyl stearate and traces of sucrose. Spray drying of the isobutanol product concentrate into supercritical carbon dioxide gave sucrose stearate powder with around 500ppm DMSO. This powder can be dissolved in ethanol again and subjected to two further sprays to reduce the DMSO content in the product to less than 0.5ppm. In this way, the FDA purity criteria are met by the final sucrose stearate product.

### Reaction variables

Sucrose to vinyl ester ratio: Theoretically, higher sucrose to vinyl ester ratio should yield higher monoester content. However, an exceedingly high ratio would create downstream purification problems. Therefore, the optimal sucrose to vinyl ester molar ratio is between about 2:1 to about 8:1.

Catalyst: This reaction is catalyzed by bases. Suitable bases include metal oxides, metal hydroxides and metal carbonates, in particularly alkali metal hydroxides and carbonates. The higher the alkalinity of the metal hydroxides and carbonates, the faster the reaction. Possible catalyst candidates include potassium hydroxide, sodium hydroxide, potassium carbonate, sodium carbonate and lithium carbonate. Other anions of weak acids that provide sufficient alkalinity are also useable.

Catalyst concentration: It was found that excessive concentration of catalyst leads to discoloration of the reaction mixture-probably due to side reactions. Therefore, the optimum catalyst concentration lies between about 1.5 and about 6g/L of total reaction volume with a potassium carbonate catalyst. When other alkali metals and/or alternate metal salts are used, the catalyst concentration should be adjusted to provide similar provide similar alkalinity.

Reaction temperature: The byproduct of using a vinyl ester as the acyl donor is acetaldehyde. Because acetaldehyde has a low boiling point, high reaction temperature is not necessary to drive the reaction as is the case with conventional methyl or ethyl esters. The minimum temperature for reasonable reaction rate is about 30°C. Although the reaction rate increases with increasing reaction temperature, high reaction temperatures are not favored because they result in discoloration of the reaction mixture, even at low catalyst concentration. A trade off between rate and discoloration (side reactions) is reached at the moderate reaction temperatures of between about 40°C and 60°C.

The following claims are thus to be understood to include what is specifically illustrated and described above, what is conceptually equivalent, what can be obviously substituted and also what essentially incorporates the essential idea of the invention. Those skilled in the art will appreciate that various adaptations and modifications of the just-described preferred embodiment can be configured without departing from the scope of the invention. The illustrated embodiment has been set forth only for the purposes of example and that should not be taken as limiting the invention. Therefore, it is to be understood that, within the scope of the appended claims, the invention may be practiced other than as specifically described herein.

## Claims

1. A process for manufacturing fatty acid sugar esters comprising the steps of:
- dissolving a sugar in an organic solvent selected from the group consisting of dimethyl sulfoxide, methyl ethyl ketone, ethyl acetate, and isobutanol to form a sugar-solvent mixture;
- mixing an alkali metal salt catalyst into the sugar solvent mixture;
- mixing a vinyl ester of a fatty acid into the sugar solvent-catalyst mixture;
- reacting the resulting mixture at a temperature between 30°C and 90°C for at least five minutes to yield a reaction product containing the organic solvent, the alkali metal salt catalyst, unreacted sugar, unreacted vinyl ester, sugar ester and acetaldehyde;
- forming an emulsion by adding brine whereupon the unreacted sugar and the metal carbonate catalyst dissolve into the brine;
- breaking the emulsion by adding an organic solvent after which phase separation occurs into an organic phase which contains crude sugar ester and unreacted vinyl ester; and
- spray drying the organic phase with supercritical carbon dioxide.

2. The process according to claim 1, wherein the sugar is selected from the group consisting of sucrose, ribose, mannose, glucose, fructose, xylose, arabinose, lactose, melibiose, galactose, mannose, raffinose and cellobiose.

3. The process according to claim 1, wherein the vinyl ester of the fatty acid is selected from the group consisting of vinyl esters of hexanoic, octanoic, decanoic, lauric, myristic, myristoletic, palmitic, stearic, oleic, ricinoleic, linoleic, linolenic, arachidic, eicosenoic, behenic, and erucic acids.

4. The process according to claim 1, wherein the alkali metal salt catalyst is selected from the group consisting of sodium carbonate, potassium carbonate, lithium carbonate, potassium hydroxide and sodium hydroxide.

5. The process according to claim 1, wherein the sugar and the vinyl ester are present at a molar ratio of between 2:1 and 8:1.

6. The process according to claim 1, wherein the step of reacting occurs at a temperature between 40°C and 50°C.

7. The process according to claim 1 further comprising removing acetaldehyde during the step of reacting.

8. The process according to claim 1, wherein a level of the vinyl ester of a fatty acid is monitored.

9. The process according to claim 1 further comprising a step of vacuum distillation to remove DMSO from the reaction product.

## Patentansprüche

1. Verfahren zur Herstellung von Fettsäure-Zuckerestern, umfassend die Schritte:
- Lösen eines Zuckers in einem organischen Lösungsmittel, ausgewählt aus der Gruppe bestehend aus Dimethylsulfoxid, Methylethylketon, Ethylacetat und Isobutanol, um ein Zucker-Lösungsmittelgemisch zu bilden;
- Zumischen eines Alkalimetallsalz-Katalysators in das Zucker-Lösungsmittelgemisch;
- Mischen eines Vinylesters einer Fettsäure in das Zucker-Lösungsmittel-Katalysatorgemisch;
- Umsetzen der resultierenden Mischung bei einer Temperatur zwischen 30°C und 90°C für mindestens 5 Minuten, um ein Reaktionsprodukt erhalten, welches das organische Lösungsmittel, den Alkalimetallsalz-Katalysator, nicht umgesetzten Zucker, unreagierten Vinylester, Zuckerester und Acetaldehyd enthält;
- Bilden einer Emulsion durch Zugabe von Salzlösung, so dass sich der nicht umgesetzte Zucker und der Metallkarbonatkatalysator in der Salzlösung lösen;
und zu ergeben
- Brechen der Emulsion durch Zugabe eines organischen Lösungsmittels, wonach eine Phasentrennung in eine organische Phase erfolgt, die Roh-Zuckerester und nicht umgesetzte Vinylester enthält;
- Sprühtrocknen der organischen Phase mit superkritischem Kohlendioxid.

2. Verfahren nach Anspruch 1, wobei der Zucker ausgewählt ist aus der Gruppe, bestehend aus Saccharose, Ribose, Mannose, Glucose, Fructose, Xylose, Arabinose, Lactose, Melibiose, Galaktose, Mannose, Raffinose und Cellobiose.

3. Verfahren nach Anspruch 1, worin der Vinylester der Fettsäure aus der Gruppe ausgewählt ist, bestehend aus Vinylestern von Hexansäure, Oktansäure, Dekansäure, Laurinsäure, Myristinsäure, Tetradekansäure, Palmitinsäure, Stearinsäure, Ölsäure, Rizinolsäure, Linolsäure, Linolensäure, Arachinsäure , Eicosansäure, Behensäure und Erucasäure.

4. Verfahren nach Anspruch 1, wobei der Alkalimetallsalz-Katalysator ausgewählt ist aus der Gruppe, bestehend aus Natriumcarbonat, Kaliumcarbonat, Lithiumcarbonat, Kaliumhydroxid und Natriumhydroxid.

5. Verfahren nach Anspruch 1, wobei der Zucker und der Vinylester in einem Molverhältnis von 2:1 und 8:1 vorhanden sind.

6. Verfahren nach Anspruch 1, wobei der Schritt der Umsetzung bei einer Temperatur zwischen 40°C und 50°C stattfindet,

7. Verfahren nach Anspruch 1, das ferner das Entfernen von Acetaldehyd während des Schritts des Umsetzens umfasst.

8. Verfahren gemäß Anspruch 1, wobei ein Pegel der Vinylester einer Fettsäure überwacht wird.

9. Verfahren gemäß Anspruch 1, ferner umfassend einen Schritt der Vakuumdestillation, um DMSO aus dem Reaktionsprodukt zu entfernen.

## Revendications

1. Procédé de fabrication d'esters de sucres et d'acides gras comprenant les étapes :
- de dissolution d'un sucre dans un solvant organique choisi dans le groupe constitué de diméthylsulfoxyde, méthyléthylcétone, acétate d'éthyle, et isobutanol pour former un mélange sucre-solvant ;
- de mélange d'un catalyseur de sel de métal alcalin dans le mélange sucre-solvant;
- de mélange d'un ester vinylique d'un acide gras dans le mélange sucre solvant-catalyseur ;
- de réaction du mélange résultant à une température entre 30°C et 90°C pendant au moins cinq minutes pour produire un produit de réaction contenant le solvant organique, le catalyseur de sel de métal alcalin, du sucre n'ayant pas réagi, de l'ester vinylique n'ayant pas réagi, de l'ester de sucre et de l'acétaldéhyde;
- de formation d'une émulsion par addition de saumure sur quoi le sucre n'ayant pas réagi et le catalyseur de carbonate de métal se dissolvent dans la saumure ;
- de rupture de l'émulsion par addition d'un solvant organique après quoi une séparation de phases a lieu en une phase organique qui contient de l'ester de sucre brut et de l'ester vinylique n'ayant pas réagi ; et
- de séchage par pulvérisation de la phase organique avec du dioxyde de carbone supercritique.

2. Procédé selon la revendication 1, dans lequel le sucre est choisi dans le groupe constitué de saccharose, ribose, mannose, glucose, fructose, xylose, arabinose, lactose, mélibiose, galactose, mannose, raffinose et cellobiose.

3. Procédé selon la revendication 1, dans lequel l'ester vinylique de l'acide gras est choisi dans le groupe constitué d'esters vlnyliques d'acides hexanoïque, octanoïque, décanoïque, laurique, myristique, myristolétique, palmitique, stéarique, oléique, ricinoléique, linoléique, linolénique, arachidique, eicosénoïque, béhénique, et érucique.

4. Procédé selon la revendication 1, dans lequel le catalyseur de sel de métal alcalin est choisi dans le groupe constitué de carbonate de sodium, carbonate de potassium, carbonate de lithium, hydroxyde de potassium et hydroxyde de sodium.

5. Procédé selon la revendication 1, dans lequel le sucre et l'ester vinylique sont présents à un rapport molaire entre 2:1 et 8:1.

6. Procédé selon la revendication 1, dans lequel l'étape de réaction a lieu à une température entre 40°C et 50°C.

7. Procédé selon la revendication 1, comprenant de plus l'élimination d'acétaldéhyde pendant l'étape de réaction.

8. Procédé selon la revendication 1, dans lequel une teneur de l'ester vinylique d'un acide gras est contrôlée.

9. Procédé selon la revendication 1 comprenant de plus une étape de distillation sous vide pour éliminer DMSO du produit de réaction.
